# EUROPÄISCHE PATENTSCHRIFT

(11) **EP 1 758 463 B1**
(45) Veröffentlichungstag und Bekanntmachung des Hinweises auf die Patenterteilung: **26.12.2007**
(21) Anmeldenummer: 05747802.6
(22) Anmeldetag: 07.06.2005
(51) Int. Cl.: A23K 1/16, A23K 1/18

(54) **GUANIDINOESSIGSÄURE ALS FUTTERMITTELZUSATZ**
GUANIDINO ACETIC ACID USED AS AN ANIMAL FOOD ADDITIVE
ACIDE GUANIDINOACETIQUE SERVANT DE COMPLEMENT POUR ALIMENTS DESTINES AUX ANIMAUX

(30) Priorität: 09.06.2004 DE 102004028193; 11.12.2004 DE 102004059761
(43) Veröffentlichungstag der Anmeldung: 07.03.2007
(73) Patentinhaber: Evonik Degussa GmbH, 40474 Düsseldorf (DE)
(72) Erfinder: GASTNER, Thomas, 84549 Engelsberg (DE); KRIMMER, Hans-Peter, 84558 Kirchweidach (DE)
(74) Vertreter: Dey, Michael
(86) Internationale Anmeldenummer: PCT/EP2005/006110
(87) Internationale Veröffentlichungsnummer: WO 2005/120246

(56) Entgegenhaltungen:
- DD-A1- 206 735
- US-A- 3 089 771

## Beschreibung

Gegenstand der vorliegenden Erfindung ist die Verwendung von Guanidinoessigsäure oder von Guanidinoessigsäure-Salzen als Futtermittelzusatz.

Guanidinoessigsäure ist eine bei Tieren und auch im Menschen vorkommende körpereigene Substanz, welche bei der Biosynthese des Kreatins eine zentrale Rolle einnimmt. Kreatin kann sowohl durch die Nahrung aufgenommen als auch endogen gebildet werden. Die Biosynthese geht von Glycin und L-Arginin aus. Bei Säugetieren wird vor allem in den Nieren, aber auch in der Leber und der Bauchspeicheldrüse, durch das Enzym Aminotransferase die Guanidino-Gruppe des L-Arginins gespalten und eine N-C-N-Gruppe auf das Glycin übertragen. Das L-Arginin wird hierbei in L-Ornithin umgewandelt. Die so gebildete Guanidinoessigsäure wird im nächsten Schritt, bei Vertebraten geschieht dies ausschließlich in der Leber, mit Hilfe des Enzyms Transmethylase in Kreatin umgewandelt. Hierbei dient das S-Adenosylmethionin als Methylgruppen-Donor. Das Kreatin diffundiert anschließend in den Blutkreislauf und wird so zu den Zielorganen transportiert. Der Transport durch die Zellmembran in die Zellen geschieht hierbei durch einen spezifischen Kreatin-Transporter.

Von Guanidinoessigsäure ist weiterhin bekannt, dass sie eine antibakterielle Wirkung besitzt und in Tierversuchen erfolgreich gegen bakterielle Infektionen (Staphyllococcus aureus) eingesetzt werden konnte (Preparation for protecting mammals against infection (Stanley Drug Products Inc., USA). Neth. Appl. (1976), 7 pp. NL 7411216).

K. Keshavarz und H. L. Fuller beschreiben im Journal of Nutrition, 101:855 - 862 (1971) den Einfluss von Arginin und Methionin auf die Kreatinbildung bei Hühnchen. Dem in diesem Zusammenhang verwendeten Basisfutter wurden unter anderem auch 1,112 % Guanidinoessigsäure (Glycocyamin) zugesetzt, was in der Folge auch in der Kombination mit Methionin zu einer deutlichen Abnahme des Gewichtszuwachses und der Futterverwertung geführt hat.

Im Zusammenhang mit der Überdosierung von Methionin ist andererseits bekannt, dass damit verbundene negative Effekte durch die Gabe von Guanidinoessigsäure abgemildert werden können (Interrelations of choline and methionine in growth and the action of betaine in replacing them. McKittrick, D. S. Univ. of California, Berkeley, Archives of Biochemistry (1947), 15 133-55).

Das schon erwähnte Kreatin nimmt im Energiestoffwechsel der Zelle eine wichtige Rolle ein, wobei es als energiereiches Phosphokreatin neben dem Adenosintriphosphat (ATP) eine wesentliche Energiereserve des Muskels darstellt. Im Ruhezustand des Muskels kann ATP auf Kreatin eine PhosphatGruppe übertragen, wobei Phosphokreatin gebildet wird, welches dann im direkten Gleichgewicht mit ATP steht. Bei Muskelarbeit ist es von entscheidender Bedeutung, die ATP-Vorräte schnellstmöglich wieder aufzufüllen. Hierfür steht in den ersten Sekunden maximaler Muskelbelastung das Phosphokreatin zur Verfügung. Dieses kann in einer sehr schnellen Reaktion, durch das Enzym Kreatinkinase eine Phosphatgruppe auf Adenosindiphosphat übertragen und somit ATP zurückbilden. Dies wird auch als Lohmann-Reaktion bezeichnet.

Kreatin ist seit langem als geeignetes Nahungsergänzungs- und Futtermittel bekannt. Bei starker und über längere Zeit anhaltender Muskelarbeit sind die natürlich im Körper vorhandenen Kreatinvorräte rasch erschöpft. Aus diesem Grund haben sich insbesondere bei Leistungssportlern gezielte Kreatingaben positiv auf die Ausdauer und Leistungsfähigkeit ausgewirkt, wobei unerwünschte Anreicherungsprozesse im Körper oder nachteilige Abbauprodukte unbekannt sind. Der Grund hierfür ist darin zu sehen, dass Kreatin bei einer übermäßigen Zufuhr vom Körper als Kreatinin ausgeschieden wird.

Weiterhin ist bekannt, dass eine Kreatin-Supplementierung zu einer Erhöhung der Körpermasse führt. Dies ist zu Anfang auf eine vermehrte Aufnahme von Wasser in den Muskel zurückzuführen. Langfristig gesehen führt das Kreatin aber indirekt durch vermehrte Proteinsynthese oder einen verminderten Proteinkatabolismus in den Myofibrillen zu einer Erhöhung der Muskelmasse (Int J Sports Med 21 (2000), 139-145). Als Ergebnis erhält man somit eine erhöhte fettfreie Körpermasse.

Neben dem Kreatin selbst, also dem Kreatin-Monohydrat, haben sich zwischenzeitlich aber auch zahlreiche Kreatinsalze, wie das Kreatinascorbat, -citrat, -pyruvat und andere, ebenfalls als geeignete Nahrungsergänzungsmittel erwiesen. Stellvertretend seien an dieser Stelle das europäische Patent EP 894 083 und die deutsche Offenlegungsschrift DE 197 07 694 A1 als Stand der Technik genannt.

Die für den Menschen als positiv nachgewiesenen Wirkungen entfaltet Kreatin auch bei Tieren, weshalb seine Verwendung in diversen Futtermitteln ebenfalls hinlänglich vorbeschrieben ist. So ist aus der internationalen Patentanmeldung WO 00/67 590 die Verwendung von Kreatin oder von Kreatin-Salzen als Futterzusatz für Zucht- und Masttiere, als Ersatz für Fleischmehl, Fischmehl und/oder antimikrobielle Leistungsförderer, Wachstumshormone sowie Anabolika vorbeschrieben. GB 2 300 103 lehrt die Verwendung von Kreatin in Form eines Hundebisquits, wofür das Kreatin-Monohydrat gemeinsam mit Fleisch in einer extrudierten Masse angeboten wird. Da Kreatin-Monohydrat aufgrund seiner schlechten Löslichkeit aber nur unzureichend bioverfügbar ist, wird seine gemeinsame Verwendung mit weiteren physiologisch aktiven Verbindungen, vorzugsweise in Salzform, empfohlen. Die deutsche Offenlegungsschrift DE 198 36 450 A1 hat die Verwendung stabiler Brenztraubensäure-Salze und insbesondere des Kreatinpyruvats in Formulierungen zum Gegenstand, die für die Tierernährung geeignet sind.

DE 100 03 835 A1 hat Formulierungen bei Dehydratationszuständen zum Gegenstand, wie sie generell bei älteren Personen und insbesondere solchen mit eingeschränkter Mobilität auftreten. In diesem Falle fungiert Kreatin als Transportmedium für Wasser, um so den durch Dehydratationserscheinungen am stärksten betroffenen Geweben Feuchtigkeit zuzuführen.

Neben seinen unbestrittenen positiven physiologischen Eigenschaften besitzt Kreatin aber auch den Nachteil, dass es als Kreatin-Monohydrat in den entsprechenden wässrigen Lösungen keine ausgeprägte Stabilität besitzt, wobei es sich über längere Zeit in Kreatinin umwandelt. Dies ist vor allem in sauren Lösungen ein Problem und ist somit auch für die orale Aufnahme und Bioverfügbarkeit von Kreatin von Bedeutung. Der pH-Wert des Magens von 1 bis 2 kann je nach Verweilzeit zu einem deutlichen Abbau des Kreatins zu Kreatinin führen. So konnte beim Menschen gezeigt werden, dass nach einer oralen Applikation von Kreatin nur etwa 15 bis 30% von der Muskulatur resorbiert werden können (Greenhaff, P.L.: Factors Modifying Creatine Accumulation in Human Skeletal Muscle. In: Creatine. From Basic Science to Clinical Application. Medical Science Symposia Series Volume 14, 2000, 75-82).

Abschließend sei noch auf den Beitrag von John W. Poutsiaka (Department of Biology Fordham University New York; in: American Journal of Physiology) hingewiesen. In diesem Artikel aus dem Jahr 1956 wird der Einfluss von Folacin, Vitamin B₁₂ und methylierenden Verbindungen auch in Gegenwart von Guanidinoessigsäure auf das Wachstum und den Muskelkreatinspiegel bei jungen Ratten beschrieben. Bezüglich des Wachstums wird der Guanidinoessigsäure in diesem Artikel eine hemmende Wirkung zugeschrieben. Auf den Muskelkreatinspiegel übte Guanidinoessigsäure keinerlei Einfluss aus, wenn es in Kombination mit Folacin und Vitamin B₁₂ verabreicht wurde. Im Falle der zusätzlichen Verabreichung von Methionin nahm der Kreatingehalt in der Skelettmuskulatur und im Herzmuskel zu. Auf Grund dieser Beobachtungen wurde daraus geschlossen, dass die Guanidinoessigsäure im Körper aus Arginin und Glycin gebildet wird, die beide als Aminosäuren vorrangig für das Methionin-gestützte Wachstum verantwortlich sind.

Aus den bzgl. Kreatin geschilderten Nachteilen des Standes der Technik hat sich für die vorliegende Erfindung die Aufgabe gestellt, Verbindungen zu finden, welche als Futtermittel oder Futtermittelzusatz für Zucht- und Masttiere verwendet werden können und sich positiv auf die Verbesserung der Futteraufnahme, die Steigerung der Mastleistung, die Erhöhung des Muskelfleischansatzes, die Fleischqualität und/oder die Reproduktionsleistung auswirken. Die Verbindungen sollten eine möglichst geringe Instabilität, insbesondere in wässriger Lösung aufweisen und bevorzugt erst nach Applikation bzw. physiologischer Aufnahme in Kreatin umgewandelt werden. Die eingesetzten Futtermittel bzw. Futterzusatzmittel sollten selbst keine physiologisch nachteiligen Wirkungen entfalten und leicht nachweisbar sein. Unter kommerziellen Gesichtspunkten stand für die erfindungsgemäß zu verwendenden Substanzen mit im Vordergrund, dass diese auf wirtschaftlich günstige Weise herstellbar sein sollten.

Gelöst wurde diese Aufgabe durch die Verwendung von Guanidinoessigsäure und/oder Guanidinoessigsäure-Salzen als Futtermittelzusatz für Zucht- und Masttiere in vorwiegend vegetarischen Diäten zur Verbesserung der Futteraufnahme, zur Steigerung der Mastleistung, des Muskelfleischansatzes, der Fleischqualität und/oder der Reproduktionsleistung.

Der hierin verwendete Begriff "vorwiegend vegetarische Diät" beschreibt eine Diät, welche vorzugsweise in Übereinstimmung mit den gesetzlichen Richtlinien in der Europäischen Union keine tierischen Bestandteile enthält. Ausgenommen ist hierbei nur ein möglicher Zusatz von Fischmehl. Weiterhin ist unter einer "vorwiegend vegetarischen Diät" gemäß dieser Erfindung auch ein Teilersatz von Fischmehl oder Fleischmehl durch Guanidinoessigsäure zu verstehen.

Überraschend wurde bei der erfindungsgemäßen Verwendung festgestellt, dass die beanspruchten Verbindungen tatsächlich die gemäß Aufgabenstellung gewünschten Eigenschaften aufweisen, da sie in einfacher und wirtschaftlicher Weise herzustellen sind, beispielsweise durch Verfahren wie die Umsetzung von Glycin und Cyanamid in wässrigen Lösungen (Production of guanidino fatty acids (Vassel, Bruno; Janssens, Walter D.)(1952), US 2,620,354; Method of preparation of guanidino fatty acids (Vassel, Bruno; Garst, Roger)(1953), 5pp. US 2,654,779).

Im Gegensatz zu Kreatin bzw. Kreatin-Monohydrat weisen Guanidinoessigsäure und deren Salze zudem in saurer wässriger Lösung eine deutlich höhere Stabilität auf und sie werden erst unter physiologischen Bedingungen in Kreatin umgewandelt. Überraschenderweise hat sich als besonders vorteilhaft herausgestellt, dass die erfindungsgemäß verwendete Guanidinoessigsäure und deren Salze im Gegensatz zum Kreatin tatsächlich erst nach der Resorption, vor allem in der Leber, in Kreatin umgewandelt werden. Somit wird im Gegensatz zum Kreatin der überwiegende Teil der verabreichten bzw. gefütterten Verbindungen, Guanidinoessigsäure und/oder Salze der Guanidinoessigsäure, nicht durch Instabilitätsreaktionen, z.B. im Magen, abgebaut und vor der Resorption ausgeschieden, sondern steht tatsächlich bei den entsprechenden physiologischen Stoffwechselreaktionen zur Verfügung.

Die Guanidinoessigsäure und deren Salze können gemäß Erfindung, somit wiederum im Gegensatz zum Kreatin und dessen Derivaten, bei identischer Wirkung mit deutlich geringerer Dosierung eingesetzt werden. Die Vorteile der mit der Erfindung beanspruchten Verwendung waren in ihrer Gesamtheit insbesondere deshalb so nicht vorherzusehen, da Guanidinoessigsäure beispielsweise bei Hühnchen eine negative Wirkung auf die Futterverwertung und dem Gewichtszuwachs zugeschrieben wurde.

Die beanspruchte Verwendung von Guanidinoessigsäure und deren Salzen als Futtermittelzusatz hat sich beispielsweise für Geflügel, wie bspw. Hühner, Puten, Enten und Gänse, aber auch für Schweine als sehr effektiv erwiesen.

Die vorliegende Erfindung sieht in einer anderen Ausführungsform vor, Guanidinoessigsäure und/oder deren geeignete Salze ergänzend bzw. alternativ als Futtermittelzusatz in Aquakulturen, vorzugsweise als Teil- oder Totalersatz für Fischmehl und/oder antimikrobielle Leistungsförderer einzusetzen, wobei die vorgeschlagene Verwendung für lachsartige (Salmoniden) und garnelenartige (Natania) bevorzugt ist.

Antimikrobielle Leistungsförderer sind Substanzen wie beispielsweise Carbadox, Olaquindox, Salinomycin, Monensin, Avilamycin oder Flavomycin. Diese werden insbesondere zur Verhinderung der Ausbreitung von Krankheiten bei Tieren eingesetzt. Weiterhin soll eine höhere Effizienz in der tierischen Produktion erzielt werden. Antimikrobielle Leistungsförderer werden auch zur Vermeidung der Übertragung von Zoonosen auf den Menschen verwendet und ermöglichen somit die Produktion qualitativ hochwertiger und sicherer tierischer Lebensmittel.

Die vorliegende Erfindung betrifft auch die Verwendung von Guanidinoessigsäure und/oder deren Salzen zur Herstellung eines therapeutischen Mittels für Zucht- und Masttiere, welches zur Stärkung des Immunsystems und zur Verbesserung der Reproduktionsleistung eingesetzt werden kann.

Das beschriebene therapeutische Mittel kommt in einer bevorzugten Ausführungsform vorzugsweise bei Geflügel und/oder Schweinen zum Einsatz.

Für die Zwecke der vorliegenden Erfindung sind prinzipiell alle Guanidinoessigsäuresalz geeignet, welche ernnährungsphysiologisch akzeptabel sind. Für die erfindungsgemäße Verwendung haben sich insbesondere Guanidinoessigsäuresalze als günstig erwiesen, die mit Salzsäure, Bromwasserstoffsäure und Phosphorsäure erhalten werden. Es können auch Mischungen aus Guanidinoessigsäure mit einem oder mehreren dieser Salze oder aber auch Mischungen der Salze untereinander eingesetzt werden.

Als weiterer Vorteil der Verwendung gemäß Erfindung hat sich herausgestellt, dass die Guanidinoessigsäure und deren Salze in einem breiten Dosierbereich eingesetzt werden können. Tagesdosen liegen bei Hühnern pro kg Lebendmasse beispielsweise im Bereich zwischen etwa 10 mg und etwa 1200 mg, insbesondere im Bereich von etwa 50 mg bis etwa 250 mg. Einzeldosen liegen im Allgemeinen im Bereich von etwa 10 mg und etwa 600 mg, bevorzugt im Bereich von etwa 25 bis etwa 125 mg. Bei Schweinen liegen Tagesdosen pro kg Lebendmasse beispielsweise im Bereich von etwa 10 mg und etwa 1000 mg, insbesondere im Bereich von etwa 25 mg bis etwa 150 mg. Einzeldosen liegen im Allgemeinen im Bereich von etwa 10 mg und etwa 500 mg, bevorzugt im Bereich von etwa 10 mg bis etwa 500 mg, bevorzugt im Bereich von etwa 10 bis 100 mg.

Im Hinblick auf die beschriebene Verwendung als Futtermittelzusatz kommen je nach Tierspezies vorzugsweise Dosen von etwa 0,01 bis etwa 100 g/kg Futtermittel oder therapeutischem Mittel in Frage, wobei Mengen von etwa 1,0 bis etwa 5,0 g als besonders bevorzugt anzusehen sind.

Da die beanspruchte Verwendung bevorzugt im nicht-veterinärmedizinischen Anwendungsbereich vorgenommen wird, haben sich Applikationsformen von Futtermittelzusätzen als besonders geeignet erwiesen, die Pulver, Granulate, Pastillen, Kapseln, Pellets oder Gelee-(Hydrokolloid-)Produkte darstellen. Dabei ist es in Abhängigkeit vom jeweiligen konkreten Verwendungsfall bevorzugt, die Guanidinoessigsäure und deren Salze als Futtermittelzusatz in Kombination mit anderen physiologisch aktiven Wirkstoffen einzusetzen, wobei insbesondere Kohlenhydrate, Fette, Aminosäuren (z.B. Kreatin), Proteine, Vitamine, Mineralstoffe, Spurenelemente und deren Derivate und beliebige Mischungen daraus besonders geeignet sind. Als bevorzugt gelten Methionin, Betain und Cholin sowie andere physiologisch wirksame Methylgruppen-Donoren. Betain und Cholin können in Gegenwart von Homocystein im Körper zu Methionin umgewandelt werden, was v.a. eine Rolle bei der Synthese von Kreatin aus Guanidinoessigsäure spielt. Hier werden Methylgruppen benötigt, die unter Bildung von Homocystein aus S-Adenosylmethionin übertragen werden. Stehen Betain oder Cholin nicht ausreichend zur Verfügung, wird Methionin verbraucht und es kann zu einem Methionin-Defizit im Stoffwechsel kommen.

Die Sterblichkeit von Zucht- und Masttieren aufgrund von erhöhten Umgebungstemperaturen stellt in vielen Ländern, vor allem im Sommer, ein Problem dar. Im Rahmen dieser Erfindung konnte überraschenderweise gezeigt werden, dass eine Supplementation mit Guanidinoessigsäure oder deren Salzen zu einer Abmilderung der Folgen von Hitzestress, insbesondere zur Vorbeugung oder Verringerung der Sterblichkeit dieser Tiere bei Hitzestress führt, d.h. beispielsweise zu einer Verringerung der Sterblichkeit in Folge erhöhter Umgebungs-Temperaturen. Es wird angenommen, dass dieser Effekt auf das aus Guanidinoessigsäure gebildete Kreatin zurückzuführen ist, welches zu einer verbesserten Versorgung der betroffenen Gewebe mit Wasser führt. Ähnliche Effekte konnten auch schon bei der Verwendung von Glycin beobachtet werden (US 2004 0043105 A1).

Ein weiterer Aspekt der vorliegenden Erfindung ist somit die Verwendung von Guanidinoessigsäure und/oder deren Salzen zur Herstellung eines Mittels für Zucht- und Masttiere zur Vorbeugung und Abmilderung der Folgen von Hitzestress, insbesondere zur Verringerung der Sterblichkeit infolge von erhöhten Umgebungstemperaturen. In diesem Falle sieht die Erfindung insbesondere vor, dass das therapeutische Mittel in vorwiegend vegetarischen Diäten als Teil- und/oder Totalersatz für Fischmehl, Fleischmehl, Anabolika (z.B. Stilbene, Steroide, Thyreostatika und β-Agonisten), antimikrobielle Leistungsförderer und/oder Wachstumshormone dient.

Weiterhin können Guanidinoessigsäure und deren Salze als Futtermittelzusatz für Nass- und Trockenfutter für Hunde und Katzen verwendet werden, wobei sich positive Auswirkungen auf das Immunsystem und den Allgemeinzustand der Tiere ergeben.

Insgesamt werden mit der vorliegenden Erfindung Guanidinoessigsäure und deren Salze neuen Verwendungszwecken als Futtermittel bzw. Futtermittelzusatz in der Ernährung von Zucht- und Masttieren oder als therapeutische Mittel für Zucht- und Masttiere zugeführt, welche im Gegensatz zu den bislang verwendeten Kreatin-Verbindungen deutliche und überraschende Vorteile aufweisen. Die nachfolgenden Beispiele veranschaulichen weiterhin die vorliegende Erfindung.

### Beispiele

1. Erfindungsbeispiele
   1.1 Eine Formulierung bestehend aus 5.000 mg Guanidinoessigsäure und 5.000 mg Insulin wurde in eine typische Rezeptur für Futterpellets zur Futterergänzung von Pferden eingebracht.
   1.2 Eine Formulierung bestehend aus 7.000 mg Guanidinoessigsäure und 750 mg Carnitintartrat wurde in die Basismasse für Lachsfutter eingebracht.
   1.3 Als Grundmischung wurde in handelsübliches Schweinefutter folgende
      Formulierung homogen eingebracht: 3.000 mg
      Guanidinoessigsäurephosphat, 3.000 mg Kreatin, 40 mg
      Magnesiumstearat, 25 mg Carboxymethylcellulose und 135 mg Lactose.
   1.4 Futtermittel für Masthühner
      Es wurde festgestellt, dass durch den Zusatz von 0,092 Gew.-% Guanidinoessigsäure (0,92 g/kg) zum luftgetrockneten Futter bei 42 Tagen Mastdauer eine Steigerung des Endgewichts um 7 % gegenüber bisherigen Fütterungsmethoden ohne Guanidinoessigsäure erzielt wurde. Dieser Zuwachs an Gewicht wurde ausschließlich durch Fleischzunahme, jedoch nicht durch Fettzunahme bzw. eine Wassereinlagerung erreicht (Verbesserung des Lean-Body-Mass-Index), wobei das Fleisch auch eine verbesserte Qualität aufwies. Zusätzlich ging mit diesem Futtermittelzusatz der Futtermittelverbrauch um etwa 6 % gegenüber bisherigen Fütterungsmethoden zurück.
      Weiterhin konnte in diesem Versuch gezeigt werden, dass bereits ein Zusatz von 0,032 Gew.-% Guanidinoessigsäure (0,32 g/kg) zum luftgetrockneten Futter bei 42 Tagen Mastdauer das Endgewicht um 3 % erhöht. Der Futterverbrauch ging um 3 % gegenüber bisherigen Fütterungsmethoden zurück. Bei der Kontrollgruppe mit einem Zusatz von 0,04 Gew.-% Kreatin-Monohydrat (0,4 g/kg) zum luftgetrockneten Futter bei 42 Tagen Mastdauer konnte hingegen keine Steigerung des Endgewichts und kein verringerter Futterverbrauch festgestellt werden.
2. Vergleichsbeispiel (gemäß EP 920 689)
   Die Auswirkung des Zusatzes von Kreatin im Futtermittel für Masthühner wurde untersucht.
   Dabei wurde festgestellt, dass durch den Zusatz von 0,2% Kreatin (0,2g/kg) zum luftgetrockneten Futter bei 41 Tagen Mastdauer eine Steigerung des Endgewichts um 4% gegenüber bisherigen Fütterungsmethoden (ohne Kreatinzusatz) erzielt wurde. Dieser Zusatz an Gewicht wurde nur durch Fleischzunahme, jedoch nicht durch Fettzunahme erreicht (Verbesserung des Lean-Body-Mass-Index), wobei das Fleisch auch eine verbesserte Qualität aufwies.
   Der Futtermittelverbrauch sank dabei um etwa 2-3% gegenüber bisherigen Fütterungsmethoden.

## Patentansprüche

1. Verwendung von Guanidinoessigsäure und/oder deren Salzen zur Herstellung eines Futtermittelzusatzes für Zucht- und Masttiere in vorwiegend vegetarischen Diäten zur Verbesserung der Futteraufnahme, zur Steigerung der Mastleistung, des Muskelfleischansatzes, der Fleischqualität und/ oder der Reproduktionsleistung.

2. Verwendung nach Anspruch 1 in Aquakulturen, vorzugsweise als Teil- oder Totalersatz für Fischmehl und/oder antimikrobielle Leistungsförderer, und insbesondere für Lachsartige (Salmoniden) und Garnelenartige (Natania).

3. Verwendung von Guanidinoessigsäure und/oder deren Salzen zur Herstellung eines therapeutischen Mittels für Zucht- und Masttiere zur Stärkung des Immunsystems und der Verbesserung der Reproduktionsleistung, wobei das therapeutische Mittel vorzugsweise in vorwiegend vegetarischen Diäten als Teil- oder Totalersatz für Fischmehl, Fleischmehl, Anabolika, antimikrobielle Leistungsförderer und/oder Wachstumshormone dient.

4. Verwendung von Guanidinoessigsäure und/oder deren Salzen zur Herstellung eines therapeutischen Mittels für Zucht- und Masttiere zur Vorbeugung und Abmilderung der Folgen von Hitzestress, insbesondere zur Verringerung der Sterblichkeit in Folge von erhöhten Umgebungstemperaturen.

5. Verwendung nach einem der Ansprüche 1,3 oder 4 für Geflügel und/oder Schweine.

6. Verwendung nach einem der Ansprüche 1 bis 5, **dadurch gekennzeichnet, dass** als Salze der Guanidinoessigsäure Salze der Salzsäure, Bromwasserstoffsäure und/oder Phosphorsäure eingesetzt werden.

7. Verwendung nach einem der Ansprüche 1 bis 6, **dadurch gekennzeichnet, dass** die Guanidinoessigsäure-Komponente in Einzeldosen von 0,01 bis 100 g und besonders bevorzugt 1,0 bis 5,0 g/kg Futtermittel oder therapeutischem Mittel eingesetzt wird.

8. Verwendung nach einem der Ansprüche 1 bis 7 in Form von Pulvern, Granulaten, Pastillen, Kapseln, Pellets, Konglomeraten oder Gelee-Produkten.

9. Verwendung nach einem der Ansprüche 1 bis 8 in Kombination mit anderen physiologisch aktiven Wirkstoffen, ausgewählt aus Kohlenhydraten, Fetten, Aminosäuren, Proteinen, Vitaminen, Mineralstoffen und/oder Spurenelementen, insbesondere Methionin, Betain, Cholin und anderen physiologisch wirksamen Methylgruppen-Donoren, sowie deren Derivaten und Mischungen daraus.

10. Verwendung von Guanidinoessigsäure und/oder deren Salzen zur Herstellung eines Futtermittelzusatzes für Nass- und Trockenfutter für Hunde und Katzen zur Verbesserung des Immunsystems oder/und des Allgemeinzustandes der Tiere.

## Claims

1. The use of guanidinoacetic acid and/or salts thereof for the production of a feed additive for breeding and growing livestock in predominantly vegetarian diets to improve feed intake, to increase growth performance, muscle meat gain, meat quality and/or reproductive performance.

2. The use as claimed in claim 1 in aquacultures, preferably as partial or total replacement of fish meal and/or antimicrobial performance enhancers, and in particular for salmonides and Natania.

3. The use of guanidinoacetic acid and/or salts thereof for the production of a therapeutic composition for breeding and growing livestock to strengthen the immune system and to improve the reproductive performance, the therapeutic composition preferably serving, in predominantly vegetarian diets, as partial or total replacement for fish meal, meat meal, anabolic agents, antimicrobial performance enhancers and/or growth hormones.

4. The use of guanidinoacetic acid and/or salts thereof for the production of a therapeutic composition for breeding and growing livestock for the prevention and mitigation of the consequences of heat stress, in particular for reducing mortality as a consequence of elevated ambient temperatures.

5. The use as claimed in one of claims 1, 3 or 4 for poultry and/or pigs.

6. The use as claimed in one of claims 1 to 5, **characterized in that**, as salts of guanidinoacetic acid, use is made of salts of hydrochloric acid, hydrobromic acid and/or phosphoric acid.

7. The use as claimed in one of claims 1 to 6, **characterized in that** the guanidinoacetic acid component is used in individual doses from 0.01 to 100 g, and particularly preferably 1.0 to 5.0 g/kg of feed or therapeutic composition.

8. The use as claimed in one of claims 1 to 7 in the form of powders, granules, pastilles, capsules, pellets, conglomerates or gel products.

9. The use as claimed in one of claims 1 to 8 in combination with other physiologically active compounds selected from carbohydrates, fats, amino acids, proteins, vitamins, minerals and/or trace elements, in particular methionine, betaine, choline and other physiologically active methyl group donors, and also derivatives thereof and mixtures thereof.

10. The use of guanidinoacetic and/or salts thereof for the production of a feed additive for wet and dry feeds for dogs and cats for improving the immune system or/and the general condition of animals.

## Revendications

1. Utilisation de l'acide guanidinoacétique et/ou de ses sels pour la préparation d'un additif d'aliment pour animaux destiné à des animaux destinés à la reproduction et à l'engraissement dans des régimes principalement végétariens pour améliorer l'assimilation des aliments, augmenter le rendement d'engraissement, la quantité de tissu musculaire, la qualité de la viande et/ou la performance de reproduction.

2. Utilisation selon la revendication 1 dans l'aquaculture, de préférence en tant que produit de remplacement total ou partiel de la farine de poisson et/ou en tant que stimulant d'activité antimicrobienne, et en particulier pour des variétés de saumons (salmonidés) et des variétés de crevettes (Natania).

3. Utilisation de l'acide guanidinoacétique et/ou de ses sels pour la préparation d'un agent thérapeutique destiné à des animaux destinés à la reproduction et à l'engraissement pour renforcer le système immunitaire et améliorer la performance de reproduction, l'agent thérapeutique étant de préférence utilisé dans des régimes principalement végétariens comme produit de remplacement total ou partiel de la farine de poisson, de la farine de viande, des anabolisants, des stimulants d'activité antimicrobienne et/ou des hormones de croissance.

4. Utilisation de l'acide guanidinoacétique et/ou de ses sels pour la préparation d'un agent thérapeutique destiné à des animaux destinés à la reproduction et à l'engraissement pour prévenir et atténuer les conséquences d'un stress thermique, en particulier pour abaisser la mortalité due à des températures élevées de l'environnement.

5. Utilisation selon l'une des revendications 1, 3 ou 4 destinée à la volaille et/ou aux porcs.

6. Utilisation selon l'une des revendications 1 à 5, **caractérisée en ce que** l'on utilise comme sels de l'acide guanidinoacétique des sels de l'acide chlorhydrique, de l'acide bromhydrique et/ou de l'acide phosphorique.

7. Utilisation selon l'une des revendications 1 à 6, **caractérisée en ce que** l'on utilise le composant acide guanidinoacétique à des doses unitaires de 0,01 à 100 g et de façon particulièrement préférée de 1,0 à 5,0 g/kg d'aliment pour animaux ou d'agent thérapeutique.

8. Utilisation selon l'une des revendications 1 à 7 sous forme de poudres, de granulés, de pastilles, de capsules, de pellets, d'agglomérés ou de produits en gelée.

9. Utilisation selon l'une des revendications 1 à 8 en combinaison avec d'autres substances physiologiquement actives, choisies parmi des hydrates de carbone, des matières grasses, des aminoacides, des protéines, des vitamines, des minéraux et/ou des oligoéléments, en particulier la méthionine, la bétaïne, la choline et d'autres donneurs de groupes méthyle physiologiquement actifs, et leurs dérivés et leurs mélanges.

10. Utilisation de l'acide guanidinoacétique et/ou de ses sels pour la production d'un additif alimentaire pour des aliments humides et secs destinés à des chiens et des chats pour l'amélioration du système immunitaire et/ou de l'état général des animaux.
